# EUROPEAN PATENT APPLICATION

(11) **EP 4 666 982 A1**
(43) Date of publication of application: **24.12.2025**
(21) Application number: 24183673.3
(22) Date of filing: 21.06.2024
(51) Int. Cl.: A61C 7/10

(54) **ORTHOPAEDIC AND/OR ORTHODONTIC ACTUATOR**

(71) Applicant: Vandenhoekconsulting AG, 3005 Bern (CH)
(72) Inventor: van den Hoek, Jasper, 3005 Bern (CH)
(74) Representative: De Vries & Metman

(57) **Abstract**

An orthopaedic and/or orthodontic actuator is described comprising a first element (1) attachable to a first implant section (101), a second element (2) attachable to a second implant section (102), and a motion guide (3) coupling the first and second elements (1, 2) and an actuator configured to control a relative movement of the first and second elements (1,2) from a first position and/or orientation to a second position and/or orientation along a predefined path, wherein the actuator comprises first and second magnets cooperating to urge the first and second elements along the predefined path.

## Description

### Technical Field

The present disclosure relates to an orthopaedic actuator, a set of the orthopaedic actuator and an adjustment tool and a use of the orthopaedic actuator.

### Background

Correction of orthopaedic or orthodontic malpositioning of bones or teeth is very complex and time consuming. Most of the correction measures require the use and/or implantation of complicated correction devices that require high maintenance and continued readjusting of the device. A device for correcting orthopaedic and/or orthodontic malpositioning simplifying construction and/or use of such devices, and/or overcoming disadvantages of the prior art is therefore desired.

### Summary

In view of the preceding, herewith an actuator for orthopaedic and/or orthodontic corrections as set forth herein is provided. A set of the actuator and an adjustment tool is provided as well.

The actuator comprises a first element attachable or attached to a first implant section and a second element attachable or attached to a second implant section.

The first and/or second implant section might for example be a tooth or bone section, to which the actuator is attached by first or second fixation points respectively. The first and/or second implant section might for example be an orthodontic section. Also or alternatively, the first and/or second implant section might for example be a foreign body implanted into a tooth and/or bone section, e.g. (at least part of) a bone anchor, a screw, an artificial orthopaedic and/or dental implant, and/or any combination thereof.

The actuator might comprise one or more first fixation points with which the first element is attachable to the first implant section, and/or one or more second fixation points with which the second element is attachable to the second implant section. A fixation point might be formed to correspond to an anatomic feature of a (prospective) user, e.g. a brace portion fitting one or more teeth.

The first element and the second element are coupled via a motion guide, which may be a linear motion guide. Advantageously, the first element comprises the guide, respectively advantageously, at least part of the guide is part of the first element. Further advantageously, the first element and the motion guide are formed in one piece. The motion guide is designed to permit a movement of the first element relative to the second element. In particular, the motion guide is configured to control a relative movement of the first and second elements from a first position and/or orientation to a second position and/or orientation, which may preferably amount to a movement along a predefined path from the first to the second position and/or orientation; in particular this may amount to control of relative positions and/or orientations of the first and second elements along the entire range of relative motion of the first and second elements permitted by the actuator.

The movement of the first element relative to the second element allows or causes further a movement of the first fixation point relative to the second fixation point, to adjust a position and/or orientation of the first implant section relative to the second implant section, e.g. to correct a position of the first implant section towards or away from the second implant section.

The movement may at least in part be caused by a magnetic force between the first element or part thereof and the second element or part thereof. The actuator may comprise first and second cooperating magnets providing a magnetic force to urge the first and second elements along at least part of the motion guide and/or the predetermined path, to cause at least part of the movement. One or more further magnets may be provided as well. In the actuator the cooperating magnets preferably are arranged pairwise and such that their respective magnetic axes (N-S) are coaxial.

The movement may at least in part be caused by a spring force between the first element or part thereof and the second element or part thereof. The actuator may comprise one or more springs providing a spring force between at least part of the first element and at least part of the second element, to urge the first and second elements along at least part of the motion guide and/or the predetermined path, and/or to cause at least part of the movement.

A magnetic force between cooperating magnets - be it attracting in case of counter-pole magnets or repelling in case of same-pole magnets - depends, inter alia, strongly on separation distance between the cooperating magnets. In comparison, a small separation distance provides a strong force and a large separation distance provides a low force; the force may decay significantly non-linearly and asymptotically. A spring force tends to be linear with relaxation of the spring from a loaded configuration (e.g. compressed or tensioned) to an at least partly relaxed configuration (e.g. compressed or tensioned less). A combination of magnet force and spring force allows providing both a large force at short separation, mainly due to the magnetic force, and a significant force at large distance separation, mainly due to the spring force. This may allow use of the actuator with little to no maintenance.

The first element may comprise a first housing. Also or alternatively the second element may comprise a second housing. Then, as the case may be, the first housing may accommodate the first magnet, also or alternatively the second housing may accommodate the second magnet, and also or alternatively the first and/or second housing may accommodate at least part of one or more springs configured to urge the first and second elements along the predetermined path. The housing(s) may separate a magnet and/or a spring and surroundings, e.g. for one or more of structural, hygienic and biosafety reasons.

At least one of the first and second elements may comprise an adjuster, e.g. an adjustment screw and possibly an adjustment member such as a plate, controlling and/or defining a position and/or orientation of the first or second magnet, respectively, relative to a portion of the first or second element, respectively, and/or controlling and/or defining a position and/or orientation of one or more springs, if present, relative to a portion of the first or second element, respectively. The portion of the first or second element may be a housing thereof. In the actuator, the first element may comprise a first magnet adjuster, e.g. an adjustment screw and possibly an adjustment plate, defining a position and/or orientation of the first magnet relative to at least part of the first element, e.g. the first housing. Also or alternatively, the second element may comprise a second magnet adjuster, e.g. an adjustment screw and possibly an adjustment member such as a plate, defining a position and/or orientation of the second magnet relative to at least part of the second element, e.g. the second housing. Also or alternatively, the first and/or second element may comprise a first and/or, respectively, second spring adjuster, e.g. an adjustment screw and possibly an adjustment plate, defining a position and/or orientation of one or more springs, relative to at least part of the first element and/or, respectively, at least part of the second element, such as the first housing and/or, respectively, second housing. Then, the first magnet adjuster and, respectively, first spring adjuster may be one first adjuster, and/or, if applicable, the second magnet adjuster and, respectively, second spring adjuster may be one second adjuster.

One or more of the adjusters may allow adjusting a relative position and/or orientation of the first and second magnets and/or the spring(s) in a particular configuration of the first and second elements, e.g. a predetermined separation. The particular configuration may be for, or at least associated with, a desired configuration at a particular moment in use, such as an initial configuration and/or a final configuration of a treatment stage of orthopaedic and/or orthodontic treatment. The particular configuration may be defined at least in part by interaction with an adjustment tool configured to maintain the actuator in the particular configuration. A clamp or brace may form an adjustment tool. The actuator may comprise one or more connectors to cooperate with one or more matching connectors of the adjustment tool. E.g., a first and/or second magnet adjuster may allow reducing a separation between the first and second magnets in a predetermined configuration of the actuator, so as to define a magnetic force. Also or alternatively, a spring adjuster may tension (or: "load") the spring and/or set a particular desired spring position and/or orientation. Also or alternatively, a first and/or second magnet adjuster and a spring adjuster allow adjusting a relative contribution of the magnet force and spring force at one or more relative positions of the first and second elements; thus a relationship between (a strength of) the magnetic force and (a strength of) the spring force along the path may be determined.

In an embodiment, the magnets may cause a force in one direction, e.g. repelling the first and second elements from each other, and the spring may cause a force in an opposite direction, e.g. attracting the first and second elements to each other.

Note that a spring may have a relaxed state and may be compressed or tensioned relative to the relaxed state. In the relaxed state, the spring does not exert a spring force. However, the magnetic force decreases with increasing distance between, i.e. separation of, the magnets but does not decay to zero. Thus, in an embodiment of the actuator at least one spring may be configured to be tensioned, relaxed, and stressed (or the other way around: stressed, relaxed, and tensioned) associated with an increasing or decreasing relative separation of the first and second elements along the path. Thus, a desired relationship between force and separation distance of the first and second elements of the actuator, associated with a length of the actuator, may be provided by suitable selection and/or adjustment of the actuator's magnetic and/or spring forces. This facilitates designing and/or performing an orthopaedic and/or orthodontic treatment.

The first element may comprise an at least partly soft magnetic first housing accommodating the first magnet, and/or the second element may comprise an at least partly soft magnetic second housing accommodating the second magnet. Such housing may at least one of guide, contain, and focus at least part of the magnetic field of the first and/or, respectively, second magnet. Thus, a particular magnetic field distribution may be determined, and/or a relatively large magnetic force of the cooperating magnets may be contained in a relatively small volume. Preferably the at least partly soft magnetic first and/or second housing is formed to remain below magnetic saturation under the influence of at least the first and/or second magnets in operable position and/or orientation along the path.

The first and/or second elements may comprise at least one stop defining a particular relative position, e.g. an extreme relative position, of the first and second elements, e.g. associated with an end of the movement and/or the path, possibly plural stops defining opposite extreme relative position and/or opposite ends of the path. The stop may be adjustable to define a desired relative position.

The first and second elements may be at least partly sleeved into each other.

The first and second magnets may be arranged to provide an effective magnet force vector and the one or more springs may be arranged to provide an effective spring force vector; the effective magnet force and spring force vectors may coincide. Thus, contributions of both forces may add up while a moment of the combined forces may be prevented. The magnet force vector and/or the spring force vector may preferably coincide with a symmetry axis of the actuator.

An advantageous embodiment of the motion guide controls a linear movement, which may be along a single movement axis, which may be in particular along a longitudinal axis of a linear motion guide. Linear guidance may facilitate control orientation of the magnetic axes of the cooperating magnets and hence an effective magnetic force vector.

Advantageously, the motion guide allows a (preferably linear) motion in one axial direction. For example, a piston that enters the guide might move linearly in one axial direction within the guide.

Advantageously, the first or second element and the motion guide are formed in one piece, such that they cannot be separated without at least partial destruction.

The actuator may have two or more fixation points, advantageously having a first fixation point and a second fixation point.

Advantageously, in an intended use of the actuator attached to the first implant section, in particular with the first fixation point, and the second fixation point attached to the second implant section, in particular with the second fixation point, the force provided by the actuator causes movement of at least part of the first implant section towards or away from at least part of the second implant section. Thus relative position and/or orientation of the first and the second implant sections may be adjusted, in particular corrected. The first and the second implant sections may be, or be related to, bone structures, hence the actuator may be referred to as an orthopaedic actuator.

The actuator comprises first and second magnets cooperating to cause the magnetic force. Advantageously, the magnetic force is caused by a first magnet being a part of or being attached to the first element and a second magnet being a part of or being attached to the second element.

Advantageously, such magnetic force might be caused by attaching the magnets as counter pole-magnets or same-pole magnets to the first respective second element to produce a magnetic force between the two elements.

Advantageously, a movement of the first magnet relative to the second magnet is a linear motion.

Further advantageously, the vector of the magnetic force shows in a direction parallel to the path and/or a movement axis of the motion guide.

By means of the magnetic force, the first element and the second element are pressed apart respectively attracted to each other. Thus a distance between a first fixation point and a second fixation point of the actuator may change.

If the first and the second magnet are arranged as counter-pole magnets (e.g. North pole of one magnet facing South pole of the other magnet), the first and second element are attracted to each other. Such attractive effect may also be achieved and/or increased by use of a spring in the actuator, configured for relaxing from a tensioned state to an at least partly relaxed state. E.g. if the spring is appropriately pre-stressed (e.g. tensioned) the first and second element are attracted to each other. Thus, the distance between the first fixation point and the second fixation point may shrink in an intended use of the actuator, e.g. to adjust, and possibly correct, a distance between the first implant section and the second implant section.

If the first and the second magnet are arranged as same-pole magnets (e.g. North pole of one magnet facing North pole of the other magnet), the first and second element are repelling each other. Such repellent effect may also be achieved and/or increased by use of a spring in the actuator, configured for relaxing from a compressed state to an at least partly relaxed state. E.g. if the spring is appropriately pre-stressed (e.g. compressed) the first and second element are urged away from each other. Thus, the distance between the first fixation point and the second fixation point may extend in an intended use of the actuator e.g. to adjust, e.g. to correct, a distance between the first implant section and the second implant section.

Further advantageously, the first and/or the second magnet may be arranged as one or more rings encircling at least part of the first and/or the second element. Also or alternatively, at least part of a spring, e.g. one or more windings of a coil spring, may be arranged encircling at least part of the first and/or the second element.

In a further advantageous embodiment of the invention, the actuator can have a loaded and an at least partly relaxed state. In a loaded state, the first element and the second element are arranged such that a loading force acts against an actuator force, i.e. the magnetic and/or spring force. In an at least partly relaxed state, the loading force is at least partly released relative to the loaded state by the movement of the first element relative to the second element.

Advantageously, in an intended use of the actuator, the actuator in a loaded state is attached with the first fixation point to the first implant section and with the second fixation point to the second implant section. In use, the loading force may release over time by forcing the first and second elements from a first configuration to a second, at least partly relaxed, configuration, and/or, by forcing the first fixation point to change its position relative to the second fixation point until the actuator achieves an at least partly relaxed state. Thus, the first implant section may be adjusted, e.g. correct its position relative to the second implant section, e.g. towards or away from the second implant section.

In a further advantageous embodiment, the first element is a rigid first element such that there is a rigid connection between the first fixation point and the motion guide.

In a further advantageous embodiment, the second element is a rigid second element, such that there is a rigid connection between the second fixation point and the motion guide.

If the first and/or second element is a rigid element, this allows to directly transfer forces towards the first respectively second implant section, in particular if the actuator force (i.e. combination of magnetic force and spring force as the case may be) is a repelling force and in particular if a distance between the first fixation point and the second fixation point shall be extended by the actuator.

In a further advantageous embodiment, the magnetic force is caused by counter-pole magnets or same-pole magnets attached to the first element and to the second element respectively.

Herein, at least one of the first and second magnets may be a permanent magnet, preferably each of the first and second magnets being a permanent magnet. However, one of the first and second magnets may be a permanent magnet whereas the other one of the first and second magnets may be an at least partly magnetisable material, preferably soft-magnetic (ferromagnetic or paramagnetic) but diamagnetic may also be suitably employed.

In a further advantageous embodiment, the magnetic force might be caused by only one magnet attached to the first or second element, wherein the respective other first or second element is a magnetisable metal.

In a further advantageous embodiment of the invention, the actuator is configured to provide a movement between the first and second elements over a distance in a range of 2-10 mm, preferably in a range of 3-8 mm e.g. 4-6 mm. Thus, the movement may change the distance between the first fixation point and the second fixation point by an amount of 2-10 mm, preferably an amount of 3-8 mm e.g. 4-6 mm. In particular, in the intended use of the actuator, a release from the loaded state to the relaxed state moves the first fixation point relative to the second fixation point by a distance of 2-10 mm, preferably 3-8 mm e.g. 4-6mm apart or together, depending on whether the actuator force attracts or repels the first and the second element. The distance may be determined by a stop of the actuator. The actuator may be configured to provide, in a loaded state, an extension force of more than about 10 N, preferably more than 25 N, more preferably more than 50 N from the first implant section onto the second implant section. E.g. the magnets may be selected such that in a loaded state, an effective repellent magnetic force of more than about 10 N, preferably more than 25 N, more preferably more than 50 N is achieved urging the first and second elements away from each other. Such forces may be used e.g. to open an anatomic bone structure, e.g. a bone suture, in particular a palate suture and/or other craniofacial sutures. Such forces or may be difficult to achieve with mechanical means. Lower forces are required to move palate portions and/or other craniofacial portions and correct their relative position and/or orientation. Therefore, the actuator can deliver the force necessary for both applications described above.

In a further advantageous embodiment, the first magnet is arranged co-axial to the first element and the second magnet is arranged co-axial to the second element. A spring may also be arranged co-axial to the first element and/or the second element.

In a further advantageous embodiment, the movement direction respectively movement axis of the first fixation point relative to the second fixation point is parallel to a linear movement axis of a linear motion guide.

Further advantageously, a motion guide might be adapted to couple with the second element in a way that the section of the second element that goes into the motion guide hovers within the linear motion guide due to the magnetic force and/or spring force.

In a further advantageous embodiment, the first element comprises a cylinder element that forms at least part of the motion guide and the second element comprises a piston element. Then, a movement of the first fixation point relative to the second fixation point may be limited by a maximal piston stroke.

In a further advantageous embodiment, the first and/or the second element comprises a stop that defines an endpoint of the movement thus defining respectively limiting a minimal or maximal movement of the first element relative to the second element. The stop might be designed so that it can be opened and fastened, e.g. by means of a screw, to attach it to the first and/or second element, such that it can be attached at different positions on the first and/or second element. The stop might be moved along the respective first and/or second element to adjust an extreme position and/or maximal movement distance.

In a further advantageous embodiment, the first and second element are at least partly sleeved into each other. E.g. the first element may comprise a first element head. An optional collar may protrude axially from the first element head e.g. from a first surface of the first element head. The second element may form a sleeve element. The first element, e.g. the collar thereof, may then axially protrude into the sleeve element at a first end of the sleeve element. The motion guide may be formed by at least part of the sleeve element to guide the portion of the first element received within the sleeve element. To provide a stop, a portion, e.g. an end of (the collar of) the first element may provide a locking section cooperating with at a least part of the second element, e.g. the locking section may at least partly extend outwardly within the sleeve element. The locking section may cooperate with a portion of the sleeve element, e.g. the locking section may engage into an undercut of the sleeve element, in such a manner that a positive locking in axial direction is established between the first element and the second element.

A positive locking may prevent a separation of the first element and the second element, e.g. in axial direction facing in opposite direction of the first element head than the protruding collar.

An inner size of the sleeve element may be smaller than an outer size of the first element head, e.g. to prevent a separation of the first element and the second element in axial direction, e.g. in axial direction facing in a direction of the protruding collar. An axial travel of the first and second elements, e.g. the collar inside the sleeve element, is permitted, for enabling a relative motion, e.g. a linear motion in one axial direction.

Further advantageously, a first magnet may be arranged within, and in particular co-axially to, the collar adjacent to the first element head and a second magnet may be arranged within, and in particular co-axially arranged to, the sleeve element and they may in cooperation cause the magnetic force. Also, one or more springs may be arranged within, and in particular co-axially to, the collar adjacent to the first element head and within, and in particular co-axially arranged to, the sleeve element.

Further advantageously, the orthopaedic actuator comprises an adjuster, e.g. an adjustment screw, for defining a position and/or orientation of at least one of the magnets relative to at least part of the first and/or second. E.g. the adjustment screw may be arranged at least partly in the first element; in particular protruding with a first end through the first element head in axial direction towards the sleeve element. An optional adjustment plate cooperating with the adjustment screw may be provided for assisting a position and/or orientation of at least one of the first and second magnets and/or at least one spring. E.g. the adjustment plate may be arranged aligned to the collar between the first element head and the first magnet, in particular co-axially aligned to the collar. A first side of the adjustment plate may face a first side of the first element head. A first end of the adjustment screw may be attached to the first surface of the adjustment plate, in particular by magnetic forces. By adjusting the adjustment screw, the adjustment screw may act on the adjustment plate and therefore on the first magnet in axial direction within the collar, to adjust a distance between the first magnet and the second magnet.

In a further advantageous embodiment, the first element and/or the second element are covered with a biocompatible material, in particular with a PVD (Physical Vapor Deposition) coating.

A set of an orthopaedic actuator and an adjustment tool is also provided herewith.

The adjustment tool may be adapted to define and/or adjust a distance between the first fixation point and the second fixation point against the magnetic force.

Advantageously, the adjustment tool may be adapted to bring the actuator into a loaded state by adjusting a relative position of the first and second elements, in particular a distance between the first fixation point and the second fixation point.

A use of the actuator provide herein is also disclosed, e.g. use for an orthodontic correction measure.

Other advantageous embodiments are listed in the dependent claims as well as in the description below.

### Brief Description of the Drawings

The presently provided will be better understood and objects other than those set forth above will become apparent from the following detailed description thereof. Such description makes reference to the annexed drawings by way of example, wherein:
Fig. 1 shows a schematic drawing of an embodiment of an orthopaedic actuator;
Fig. 1a schematically shows the actuator of Fig. 1 provided with fixation points and in an intended use;
Fig. 2 shows a cross section of the actuator in Fig. 1;
Fig. 3 shows a schematic drawing of the embodiment in Fig. 1 after a movement of the first element relative to the second element;
Fig. 3a shows the actuator of Fig. 1a after a relative movement of the first and second elements;
Fig. 4 shows a cross-section of the embodiment in Fig. 3;
Fig. 5 shows a further embodiment of a linear guide for an orthodontic implant on which the magnetic or elastic actuator can be used;
Fig. 6a and Fig. 6b show a further embodiment;
Fig. 7a and Fig. 7b show a further embodiment;
Fig. 8a and Fig. 8b show a first element of a further embodiment;
Fig. 9 shows a second element of the further embodiment as shown in Figs. 8a and 8b;
Fig. 10, 10a, 10b schematically show an actuator generally similar to the actuator of Figs. 1-4 in various views (Fig. 10a, 10b in directions Xa, Xb indicated in Fig. 10);
Fig. 11 schematically shows a cross section of the actuator in Fig. 10, in plane XI indicted in Fig. 12;
Fig. 12 schematically shows a cross section of the actuator in Fig. 10, in plane XII indicted in Fig. 11 perpendicular to the cross-section plane XI of Fig. 11;
Fig. 13 schematically shows a graph of a relationship between forces and distance between parts of the first and second elements of the actuator;
Figs. 14-14b schematically show an actuator similar to Figs. 1-4, 10-12 in various views;
Figs. 15-16 schematically show cross sections of the actuator in Fig. 14-14b in two perpendicular planes;
Fig. 17 shows a schematic drawing of an embodiment of the orthopaedic actuator in use.

### Detailed Description of Embodiments

It is noted that the drawings are schematic, not necessarily to scale and that details that are not required for understanding the present invention may have been omitted. Elements that are at least substantially identical or that perform an at least substantially identical function are denoted by the same numeral, where helpful individualised with alphabetic suffixes.

Further, unless otherwise specified, terms like "detachable" and "removably connected" are intended to mean that respective parts may be disconnected essentially without damage or destruction of either part, e.g. excluding structures in which the parts are integral (e.g. welded or moulded as one piece), but including structures in which parts are attached by or as mated connectors, fasteners, releasable self-fastening features, etc. The verb "to facilitate" is intended to mean "to make easier and/or less complicated", rather than "to enable".

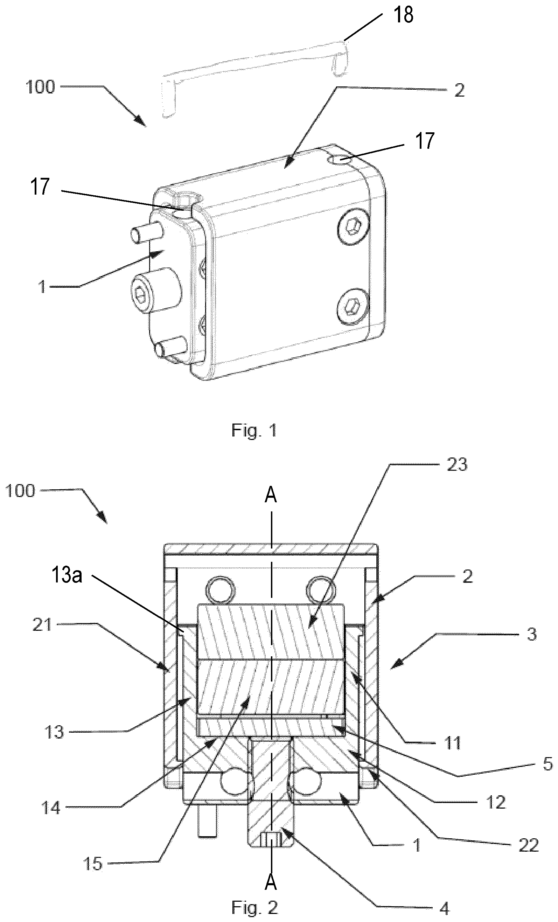

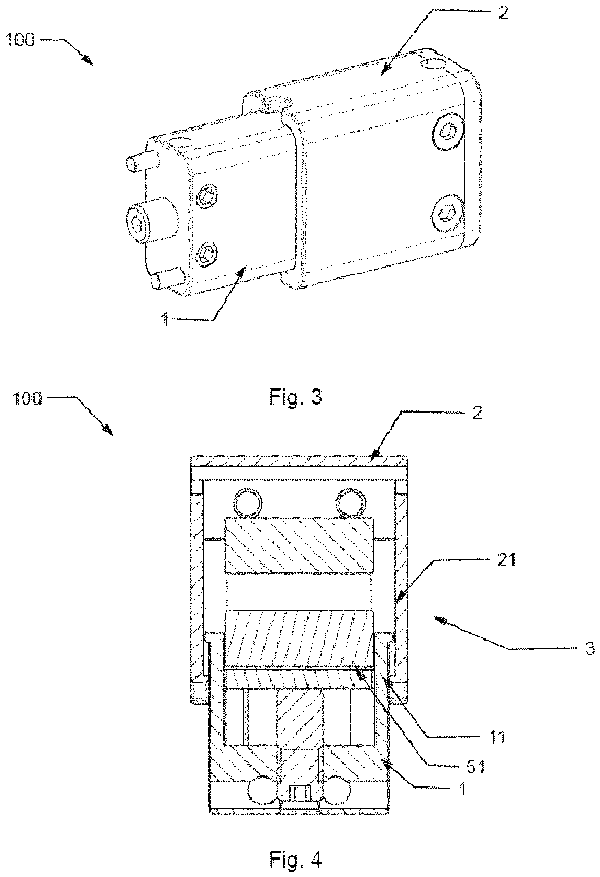

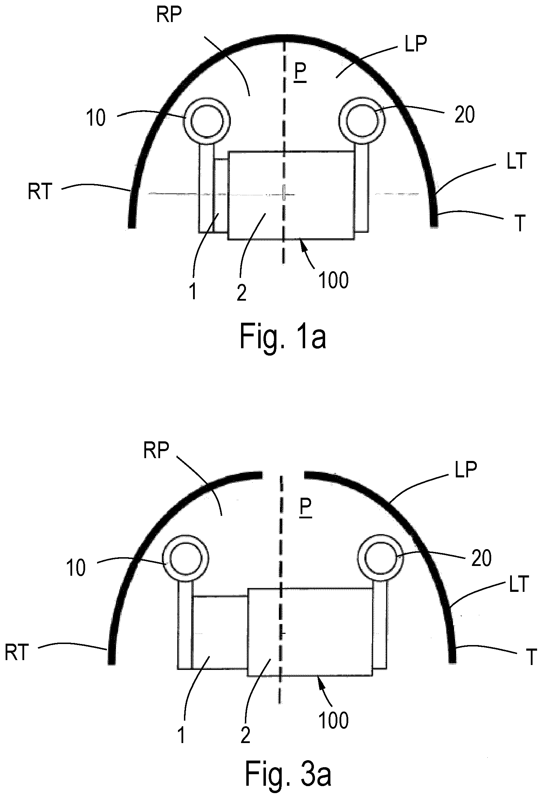

Fig. 1 to Fig. 4 show schematic drawings and cross sections of an embodiment of an actuator which may be used as orthopaedic actuator. Fig. 1 and Fig. 2 show a first state (non-extended state) of the actuator and Fig. 3 and Fig. 4 show a second state (extended state) of the actuator.

Fig. 1 shows a schematic of an embodiment. The figure depicts an orthopaedic actuator 100 which comprises a first element 1 and a second element 2. Figs. 1a and 3a show actuator 100 comprising a first element connected to a first fixation point 10 and a second element connected to a second fixation point 20.

As is visible in the cross section in Fig. 2, the first element 1 comprises further a motion guide 3, in this example a linear motion guide 3, to couple the first element 1 and the second element 2. The motion guide 3 permits a movement of the first element 1 relative to the second element 2. The motion guide 3 allows a movement of a first fixation point relative to a second fixation point.

The motion guide 3 may be provided by cooperating guiding structures of the first and second elements 1, 2, e.g. one or more portions of the first element 1 slidably received in one or more portions of the second element 2. The motion guide 3 is configured to control a relative movement of the first and second elements 1, 2 from a first to a second position and possibly from a first to a second orientation, e.g. along a predetermined path extending from the first position to the second position. The movement is caused by a magnetic force and an optional spring force between the first element 1 or parts thereof and the second element 2 or parts thereof.

As shown in the embodiments of Fig. 1 to 4, the first element 1 comprises a magnet 15 that is accommodated in a first housing and/or the second element 2 comprises a magnet 23 that is accommodated in a second housing.

The first and/or second element 1, 2 and in particular the first and/or second magnet 15, 23 may at least partly be encapsulated with a suitable encapsulation. The respective encapsulations may improve biocompatibility for human use.

In an advantageous embodiment, the first element 1 and/or the second element 2 and or the first housing and/or the second housing can comprise soft magnetic material e.g. steel parts. It is noted that stainless steel is paramagnetic. Advantageously, the soft magnetic material and magnets may be selected to prevent saturation of the soft magnetic material in normal use. In an embodiment, the first and/or second housing comprises a wall thickness w of w ≥ 0.5 mm e.g. w ≥ 0.7 mm such as w ≥ 1 mm. Further, the housing may be a steel housing with a steel having a saturation flux of Bs ≥ 1.0 T preferably Bs ≥ 1.5 T.

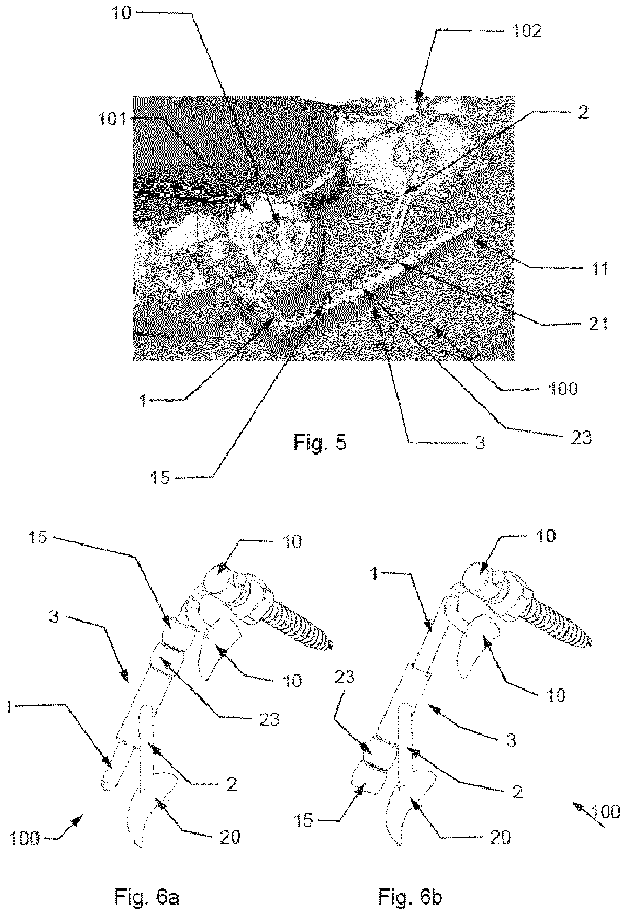

In an intended use of the actuator 100, a first fixation point 10 is attached to a first implant section 101 and a second fixation point 20 is attached to a second implant section 102 (implant sections are not visible in Fig. 1 and 2, but in Fig. 5); Figs. 1a and 3a schematically show the actuator in such use, fixed on a human palate P surrounded by a row T of teeth. The shown palate P may be divided in left and right palate portions LP, RP separated by the sutura palatina mediana (dashed line) and the teeth may be divided in left and right sets of teeth LT, RT.

Fig. 1a and Fig. 3a respectively, shows a schematic drawing of the embodiment in Fig. 1 (Fig. 1a) before and after a relative movement of the first element 1 and the second element 2, causing extension of the actuator 100.

Advantageously, the first element 1 comprises a first magnet 15 and the second element 2 comprises a second magnet 23 causing a magnetic force between the first magnet 15 and the second magnet 23 to cause the movement. The first 15 and the second 23 magnet might be same-pole or counter-pole magnets.

Advantageously, the orthopaedic actuator 100 in Figs. 1, 1a and 2 is in a loaded state, wherein the first element 1 and the second element 2 are arranged such that a loading force acts against the magnetic force.

Further advantageously, the orthopaedic actuator 100 in Figs. 3, 3a and 4 is in a comparably relaxed state, wherein the loading force is released by the relative movement of the first element 1 and the second element 2, providing extension of the actuator 100.

As indicated before, in an intended use of the orthopaedic actuator 100, an orthopaedic actuator is attached with the first fixation point 10 to the first implant section 101 and with the second fixation point 20 to the second implant section 102, in a loaded state. Then, the loading force releases over time by urging the first and second elements along a path predetermined by at least part of the actuator, thereby forcing the first fixation point 10 to change its position relative to the second fixation point 20, until the actuator achieves an at least partly relaxed state. Thus, the first implant section 101 is forced to change its position relative to the second implant section 102.

If the first and second magnets 15, 23 are counter-pole magnets they attract each other and therefore a distance between the first fixation point 10 and the second fixation point 20 decreases towards the relaxed state.

If the first and second magnets 15, 23 are same-pole magnets they repel each other and therefore a distance between the first fixation point 10 and the second fixation point 20 increases towards the relaxed state; see the differences in Figs. 1, 1A, 2 relative to Figs. 3, 3a, 4.

Advantageously, the movement changes a distance between the first fixation point 10 and the second fixation point 20 in a range of 2-10 mm, e.g. in a range of 4-6 mm.

Advantageously, as shown in Fig. 2 and Fig. 4, the first magnet 15 and the second magnet 23 are arranged co-axial to an axis A of the first element 1 and to the second element 2 respectively. The axis A may be a symmetry axis to the first and/or second element and/or actuator in general.

As shown in Fig. 2 and Fig. 4 of the advantageous embodiment, the first element 1 comprises a first element head 12. A collar 13 axially protrudes from a first surface 14 of the first element head 12.

The second element 2 forms a sleeve element 21. The collar 13 axially protrudes into the sleeve element 21 at the first end of the sleeve element 21.

The linear-motion guide 3 is comprised in the sleeve element 21, wherein an end of the collar 13 extends outwardly in the sleeve element 2 and provides a locking section 13a. The locking section 13a engages into an undercut 22 of the sleeve element 21 in such a manner, that a positive locking in axial direction is established between the first element 1 and the second element 2.

The positive-locking prevents a separation of the first element 1 and the second element 2 in axial direction facing in opposite direction of the first element head 12 than the protruding collar 13. An inner diameter of the sleeve element 21 is smaller than the outer diameter of the first element head 12 to prevent a separation of the first element 1 and the second element 2 in axial direction facing in a direction of the protruding collar 13, while an axial travel of the collar 13 inside the sleeve element 21 is permitted, for enabling the movement, respectively the linear motion in axial direction. Thus the first and second elements 1, 2 comprise a stop (13a/22) defining an end of the movement path of the first and second elements 1, 2.

Further advantageously, the orthopaedic actuator 100 comprises an adjustment screw 4. The screw 4 protrudes with a first end through the first element head 12 in axial direction towards the sleeve element 21.

Further advantageously, an adjustment plate 5 is arranged, preferably co-axially aligned to the collar 13, between the first element head 12 and the first magnet 15. Thus, a first side of the adjustment plate 5 faces a first surface of the first element head 14. The first end of the adjustment screw 4 is preferably attached to the first surface of the adjustment plate 5, in particular by magnetic forces. By adjusting the adjustment screw 4, the adjustment screw 4 acts, via the adjustment plate 5, on the first magnet 15 in axial direction within the collar 13 to adjust a distance between the first magnet 15 and the sleeve element 21, and therewith between the first magnet 15 and the second magnet 23 for a given configuration of the actuator determined by a given relative position of the first and second elements 1, 2.

Further advantageously, the adjustment plate 5 comprises one or more adjustment guides 51 to facilitate the linear movement of the adjustment plate 5 in the first element 1.

Further advantageously, the surface of the orthopaedic actuator 100 might be coated with a biocompatible coating.

Advantageously, the orthopaedic actuator 100 is used for an orthopaedic or orthodontic correction measure.

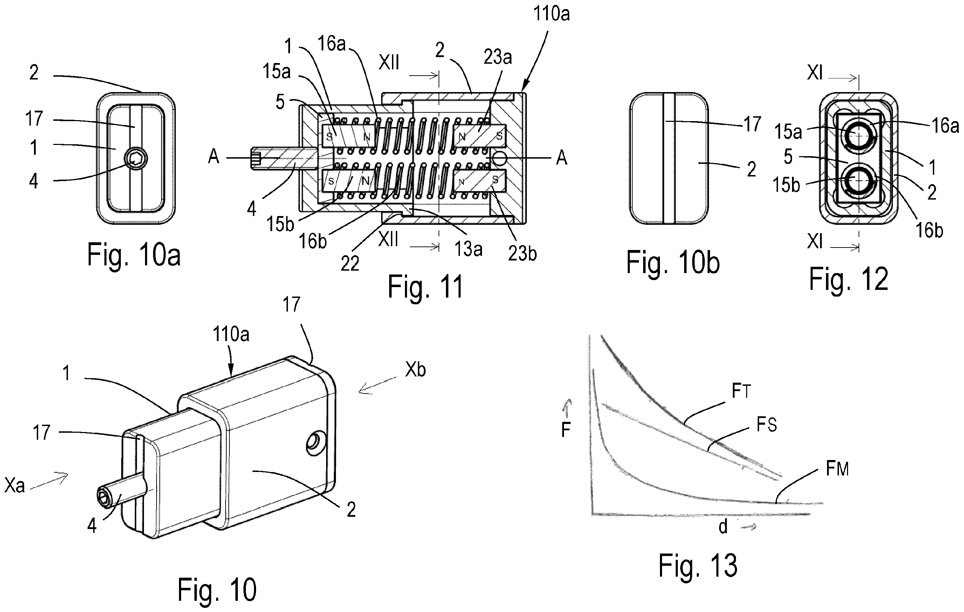

Figs. 10-12 show an actuator 110a largely comparable to the actuator 100 of Figs. 1-4. However, the actuator 110a comprises plural first magnets 15a, 15b and plural second magnets 23a, 23b, arranged pairwise opposite each other. As indicated, the magnets 15a-23a and 15b-23b may be arranged counter-poled. The magnetic force vector of the combination of all magnets may be along a symmetry axis A of the actuator 110a. Also, the actuator 110a comprises springs 16a, 16b configured to urge the first and second elements 1, 2 along the predetermined path. Here, each spring 16a, 16b is arranged coaxial with the magnets 15a-23a and 15b-23b. The spring force vector of each spring 16a, 16b may be parallel to, preferably coincide with, a magnet force vector of a set of magnets 15a, 23a / 15b, 23b associated with the respective spring 16a, 16b. The spring force vector of the combination of all springs 16a, 16b may be along the symmetry axis A of the actuator 110a, and/or coinciding with the magnetic force vector of the combination of all magnets.

One or each spring 16a, 16b may be configured to exert a spring force in the same direction as the magnets 15a-23a, 15b-23b; the spring force vector may point in the same direction as the magnetic force vector. Then, the forces may add up.

An adjuster (here again an adjustment screw 4 and an adjustment plate 5) allows adjusting a default position of at least some magnets in a particular configuration of the actuator; compare Fig. 11 with Figs. 2, 4. In Fig. 11 it may be seen that the adjuster may also adjust a length of one or more springs 16a, 16b at least in the maximally extended configuration of the actuator 110a shown in Fig. 11 (the stop 13a/22 blocks further relative outward movement of the first and second elements 1, 2). Upon compressing the actuator 110a against the magnet and spring forces, the actuator is loaded. When installed for intended use, the actuator 110a may expand and gradually relax the force associated with displacement of implant sections 101, 102 to which (the first and second fixation points 10, 20 of) the actuator 110a is (are) fixed.

To keep the loaded actuator in a loaded configuration, a keeper or other adjustment tool may be provided, e.g. a clamp and/or brace (not shown). The actuators 100 (Fig. 1) and 110a (Fig.10) are provided with optional connectors 17 for connecting such clamp 18, which may be a generally U-shaped member (Fig. 1) and/or be associated with a default state of the actuator 100, 110a.

Fig. 13 schematically shows a graph of a relationship between the magnetic force F_{M}, the spring force F_{S} and the total force F_{T} (which may be F_{T} = F_{M} + Fs) of the actuator 110a as a function of separation d of counter-pole (repellent) magnets and including a spring force urging the first and second elements 1 2, away from / out of each other, i.e. as a function of movement distance of the elements 1, 2, along the path defined by the sleeved first and second elements 1, 2. If the magnets 15, 23 are close to each other (small separation) the magnetic force F_{M} is high, and if they are far from each other (large separation the magnetic force F_{M} is low; the relationship force - distance decays strongly non-linear; precise calculation of the force at a given separation between the counter-pole magnets may be difficult. The spring force F_{S} may relate linearly with elongation of the spring, in particular for a coil spring as shown. The total force F_{T} may be a sum of the magnetic force F_{M} and the spring force Fs and exhibit both a high force at short separation and a still high force at large separation.

For separating bone structures on opposite sides of an anatomic boundary (e.g. mid-palatal suture; sutura palatina mediana; e.g. Figs. 1a, 3a) a large force is required to open the suture, whereas large distances may need to be achieved to adjust the palate sections to desired positions, for which comparably small forces may suffice. The presently provided actuator 100, 110a may be used as a platal expander and facilitate palate suture opening and palatal correction, while obviating repeated adjustment and/or specialist intervention such as repeated adjustment of a screw in known palate expanders. Hence, once a patient is suitably provided with the actuator, intervention and/or interaction with the actuator 100, 110a may be largely dispensed with.

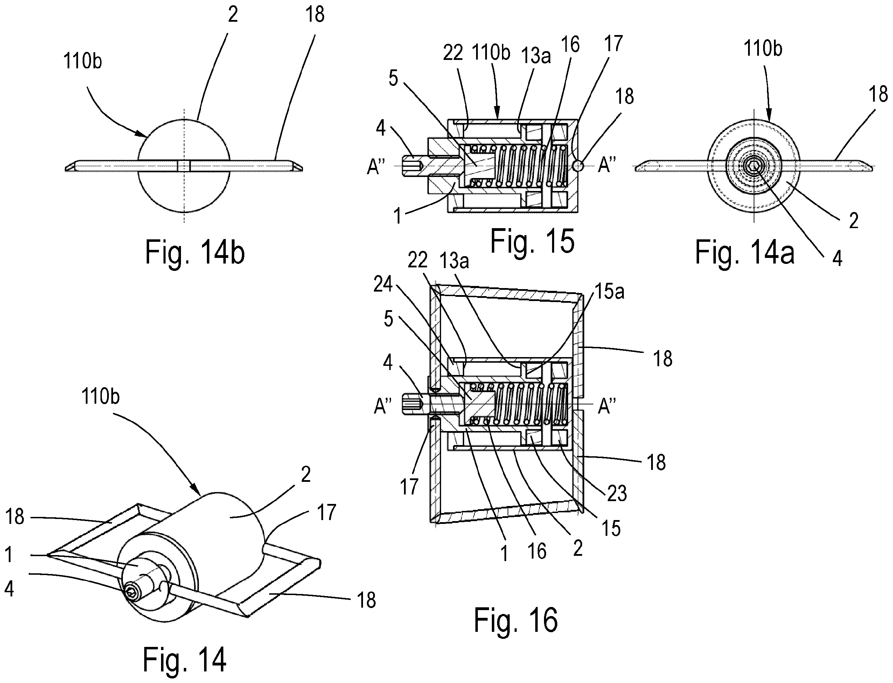

Figs. 14-16 shown another embodiment of an actuator 110b comparable to the actuator 100 and 110a of Figs. 1-4 and Figs. 10-12 (and 13) respectively, comprising a first and a second element 1, 2, sleeved into each other for movement to each other. Fixation points are not shown.

The actuator 110b is as an option largely axisymmetric about an axis A". The actuator 110b has first and a second magnet 15, 23 cooperating with each other to provide a magnet force F_{M} for urging the first and second elements 1, 2 of the actuator 110b away from / out of each other or toward / into each other, depending on their relative polarity. Here, the magnets 15, 23 are optionally formed as ring magnets wherein they may encircle at least part of the first and/or second element 1, 2. Again, the housing of at least one of the first and second elements 1, 2 preferably is/are at least partly soft magnetic to focus the magnetic fields of the magnets.

Further, the actuator 110b comprises a spring 16 accommodated in housings provided by the first and second elements 1, 2 configured to urge the first and second elements 1, 2 of the actuator 110b away from / out of each other or toward / into each other. The spring 16 is optionally formed as a coil spring (although other types and/or shapes of spring may be suitably provided in any embodiment) is as preferred option coaxially arranged about the magnets 15, 23 and/or the axis A".

In a variant of the embodiment (not shown) the actuator could be made hollow along the axis A". The actuator could be sleeved onto a device penetrating at least partly into a lumen formed in the actuator 110b, the magnets 15, 23 and spring 18 encircling the device. In such case, the adjuster (see below) could e.g. be formed as a nut or tubular screw.

The movement is guided along the axis A", e.g. by close fitting of the first and second elements 1, 2 to each other on at least two positions spaced along the axis A"; here, the first magnet 15 and/or a mounting 15a thereof forming part of the locking section 13a is fitted closely against an inner wall of the sleeve element 21 of the second element 2, and an end flange 24 - here optionally defining undercut 22 - of the second element 2 closely fits about the first element head 11 and/or further piston part of the first element 1. Thus, the housings of the first and second element 1, 2 provide a guide configured to control a relative movement of the first and second elements 1, 2.

For placing the actuator 110b in a use position, the actuator 110b may be loaded, e.g. compressed into a relatively short configuration, and provided with an optional clamp 18 to maintain the actuator 110b in the loaded configuration. Note that in another option (not shown), the actuator may be loaded to an expanded (e.g. lengthened) configuration to relax, in use, to a shorter configuration so as to move the implant sections at least partly towards each other.

Best seen in Fig. 16, in any embodiment, an adjuster 4, 5 may be configured to adjust (e.g. load) only at least part of the spring 16 of the actuator 110b; a position of one or more magnets relative to at least part of (e.g. a housing of) the first and/or second element 1, 2 may be fixed and/or unaffected by adjustment of the spring 16. A combination of one or more separate adjusters for one or more magnets and for one or more springs may be provided as well (not shown).

Further advantageously, the actuator or parts thereof is (are) covered with a bio-compatible coating, e.g. a polymeric film and/or ceramic layer.

Fig. 5 shows a further embodiment of an actuator 100 comprising a first element 1 and a second element 2. In Fig. 5, the actuator 100 is shown in the intended use for a correction measure. In particular, the first element 1 of the actuator 100 is attached to a first implant section 101, here a first tooth, with a first fixation point 10, and the second element 2 of the actuator 100 is attached to the second implant section 102, here a second tooth, with a second fixation point 20.

The first element 1 comprises a linear motion guide 3. The first element 1 and the second element 2 are coupled to each other via the linear motion guide 3. The linear motion guide 3 is designed to permit and control a movement of the first element 1 relative to the second element 2, to allow a movement of the first fixation point 10 relative to the second fixation point 20. The movement is caused by a magnetic force between the first element 1 or parts thereof and the second element 2 or parts thereof.

In an intended use of the orthopaedic actuator 100 with the first fixation point 10 attached to the first implant section 101 and the second fixation point 20 attached to the second implant section 102, the movement triggers the change of a position of the first implant section 101 towards the second implant section 102.

Further advantageously, in the intended use as shown in Fig. 5, the actuator 100 might be attached to the first 101 respectively second 102 implant section in a loaded state.

In a loaded state, the first element 1 and the second element 2 are arranged such that a loading force acts against the magnetic force.

The loading force is released over time when the magnetic force works against the current position of the first 101 and second 102 implant section until the actuator 100 reaches an extension defined by a stop and/or achieves a relaxed state.

In the relaxed state, the loading force is released by the movement of the first element 1 relative to the second element 2.

If the actuator 100 reaches an extension defined by a stop and/or achieves the relaxed state, the movement stops and the first implant section 101 and the second implant section 102 do not further change the position towards each other.

This way, the change of the distance between the first fixation point 10 and the second fixation point 20 might be controlled.

Further advantageously, the first element 1 and the second element 2 might be rigid elements to transfer the magnetic force onto the first 10 and second 20 fixation point and thus onto the first 101 and second 102 implant section.

Further advantageously, counter-pole magnets or same-pole magnets attached to the first element 1 and to the second element 2 to cause the magnetic force.

Further advantageously, the actuator or parts thereof is (are) covered with a bio-compatible film.

As an example, the first magnet 15 is arranged on the first element 1 and the second magnet 23 is arranged on the second element 2.

This arrangements of the first 15 and second 23 magnet is only exemplary. The magnets might also be designed as rings around the first 1 and second 2 element or the first 1 and/or second element 2 itself might be a magnet.

The orthopaedic actuator as shown in Fig. 1 to Fig. 5 might be used for an orthopaedic or an orthodontic correction measure.

Advantageously, one or more stopper elements 6, in particular attached to the second element 2, might limit the maximal movement of the first element 1 relative to the second element 2.

Fig. 6a and Fig. 6b show further embodiments of the orthopaedic actuator 100. These embodiments are similar to Fig. 5 but further include an orthodontic implant for anchorage. In the embodiments as shown in these figures, first magnet 15 attached to the first element 1 and second magnet 23 attached to the second element 2 are designed such that they are pronounced in the design. It is clear from the drawings that the first magnet 15 might be arranged on different positions in the first element 1. In particular, the second fixation point 20 might advantageously be designed as a screw.

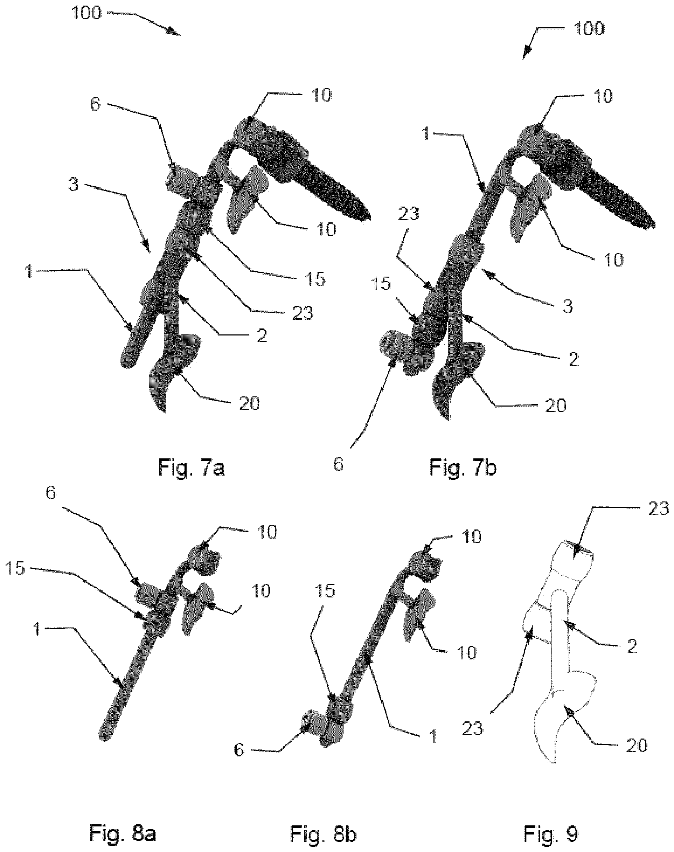

Fig. 7a and Fig. 7b show further embodiments of the orthopaedic actuator 100. In particular, the respective embodiments comprises a stopper element 6 that defines the maximal movement of the first element 1 relative to the second element 2. The stopper element 6 might be designed that it can be opened and fasted, e.g. by means of a small screw, to attach it to the first element 1, such that it can be attached at different positions on the first element 1.

Fig. 8a and 8b show further embodiments of the first element 1 comprising the multiple first fixation points 10 and the linear motion guide 3 as well as the first magnet 15. Fig. 9 shows an embodiment of the second element 2 comprising the second magnet 23.

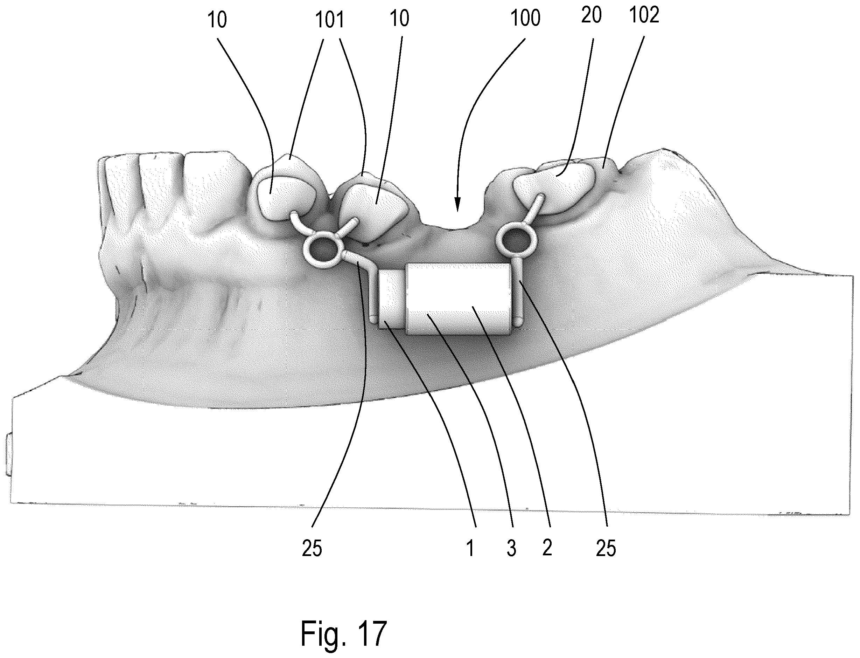

Fig. 17 shows a further embodiment of an actuator 100 comprising a first element 1 and a second element 2. In Fig. 17, the actuator 100 is shown in the intended use for a correction measure. In particular, the first element 1 of the actuator 100 is attached to the first implant section 101, here two first teeth, with two first fixation points 10, and the second element 2 of the actuator 100 is attached to the second implant section 102, here a second tooth, with a second fixation point 20. This provides a rigid structure and prevents the actuator 100 from rotating about itself.

The actuator 100 comprises a linear motion guide 3. The first element 1 and the second element 2 are coupled to each other via the linear motion guide 3. The linear motion guide 3 is designed to permit and control a movement of the first element 1 relative to the second element 2, to allow a movement of the first fixation point 10 relative to the second fixation point 20. The movement is caused by a magnetic force between the first element 1 or parts thereof and the second element 2 or parts thereof. The first element 1 and the second element 2 are fixed to the first fixation point 10 and the second fixation point 20, respectively, through rigid wires 25.

The actuator 100 as shown in Fig. 17 may also comprise one or more springs, comparable to the embodiments as shown in Figs. 10-12 and Figs. 14-16, possibly in combination with a spring adjuster, or other features related to those embodiments. A combination of magnet force and spring force allows providing both a large force at short separation, mainly due to the magnetic force, and a significant force at large distance separation, mainly due to the spring force.

Elements and aspects discussed for or in relation with a particular embodiment may be suitably combined with elements and aspects of other embodiments, in particular the subject matter of any and all appended claims may be suitably combined, unless explicitly stated otherwise.

Various examples of the concepts presented herein may be listed and/or summarised in the following clauses referring to the figures with reference symbols in parentheses:
1. An orthopaedic actuator (100) comprising
   a first element (1) attachable with a first fixation point (10) to a first implant section (101), and
   a second element (2) attachable with a second fixation point (20) to a second implant section (102),
   wherein the first element (1) and the second element (2) are coupled via a linear motion guide (3) designed to control a relative movement of the first element (1) and the second element (2), to allow a movement of the first fixation point (10) relative to the second fixation point (20) to adjust a position and/or orientation of the first implant section (101) relative to the second implant section (102),
   wherein at least part of the movement is caused by a magnetic force between at least part of the first element (1) and at least part of the second element (2).
2. The orthopaedic actuator according to clause 1 being an orthodontic implant, and/or
   wherein the first implant section (101) and/or the second implant section (102) is an orthodontic section.
3. The orthopaedic actuator according to any one of the preceding clauses,
   wherein in a loaded state the first element (1) and the second element (2) are arranged such that a loading force acts against the attracting or repelling force provided by the force unit,
   wherein in a relaxed state the loading force is at least partly released by the movement of the first element (1) relative to the second element (2),
   such that in the intended use of the orthopaedic actuator, an orthopaedic actuator in a loaded state is attached or attachable with the first fixation point (10) to the first implant section (101) and with the second fixation point (20) to the second implant section (102),
   wherein the loading force releases over time at least partly by forcing the first and second fixation points (10, 20) to change their relative position until the actuator achieves an at least partly relaxed state and for forcing the first and second implant sections (101, 102) to adjust their relative position and/or orientation.
4. The orthopaedic actuator (100) according to clause 1, wherein the first element (1) is a rigid first element (1) and/or wherein the second element (2) is a rigid second element (2).
5. The orthopaedic actuator (100) according to one of the preceding clauses, wherein counter-pole magnets or same-pole magnets coupled with the first element (1) and to the second element (2) to cause the magnetic force.
6. The orthopaedic actuator (100) according to one of the preceding clauses, wherein the force unit comprises at least one spring coupled with the first element (1) and the second element (2) to cause a spring force as at least part of the attracting or repelling force.
7. The orthopaedic actuator (100) according to one of the preceding clauses, wherein the movement changes a distance between the first fixation point and the second fixation point by 2-10 mm, in particular 4-6 mm.
8. The orthopaedic actuator (100) according to one of the preceding clauses, wherein the linear motion guide (3) permits a linear motion along solely one movement axis.
9. The orthopaedic actuator (100) according to one of the preceding clauses, wherein a first magnet is arranged co-axial to the first element (1) and a second magnet is arranged co-axial to the second element (2), wherein the attractive or repelling magnetic force between the first and the second magnet provides at least part of the attractive or repelling force.
10. The orthopaedic actuator (100) according to one of the preceding clauses, wherein a spring is arranged co-axial to the first element (1) and coaxial to the second element (2), wherein the spring provides at least part of the attractive or repelling force.
11. The orthopaedic actuator (100) according to one of the preceding clauses,
   wherein the first element (1) comprises a first element head (12), wherein a collar (13) axially protrudes from a first surface (14) of the first element head (12), and
   wherein the second element (2) forms a sleeve element (21),
   wherein the collar axially protrudes into the sleeve element (21) at a first end (22) of the sleeve element (21),
   wherein the linear-motion guide (3) is comprised in the sleeve element (21), wherein an end of the collar (13) that provides a locking section (11) extends outwardly in the sleeve element (21), which locking section (11) engages into an undercut (22) of the sleeve element (21), in such a manner that a positive locking in axial direction is established between the first element (1) and the second element (2),
   wherein the positive-locking prevents a separation of the first element (1) and the second element (2) in axial direction facing in opposite direction of the first element head (12) than the protruding collar (13),
   wherein an inner diameter of the sleeve element (21) is smaller than the outer diameter of the first element head (12) to prevent a separation of the first element (1) and the second element (2) in axial direction facing in a direction of the protruding collar (13),
   while an axial travel of the collar (13) inside the sleeve element (21) is permitted, for enabling the linear motion in one axial direction.
12. The orthopaedic actuator (100) according to clause 11, wherein a first magnet (15) arranged within and in particular co-axially to the collar adjacent to the first element head, and a second magnet (23) arranged within and in particular coaxially arranged to the sleeve element (2) cause the magnetic force.
13. The orthopaedic actuator (100) according to clause 12, comprising an an adjustment screw (4) protruding with a first end through the first element head (12) in axial direction towards the sleeve element (21),
   an adjustment plate (5) arranged co-axially aligned to the collar (13) between the first element head (12) and the first magnet (15), such that a first side of the adjustment plate (5) faces the first surface of the first element head (14),
   wherein the first end of the adjustment screw (4) is attached to the first surface of the adjustment plate (5), in particular by magnetic forces,
   such that by adjusting the adjustment screw (4), the adjustment screw (4) acts on the adjustment plate (5) and therefore on the first magnet (15) in axial direction within the collar (13) to adjust a distance between the first magnet (15) and the second magnet (23).
14. The orthopaedic actuator (100) according to one of the preceding clauses, wherein the first element (1) and the second element (2) is covered with a bio-compatible film.
15. The orthopaedic actuator (100) according to one of the preceding clauses, wherein a movement direction of the first fixation point (10) relative to the second fixation point (20) is parallel to a linear movement axis of the linear motion guide (3).
16. The orthopaedic actuator (100) according to one of the preceding clauses, wherein the first element (1) and/or the second element (2) comprises a stopper element (6) that limits the maximal movement of the first element (1) relative to the second element (2).
17. Set of an orthopaedic actuator (100) according to one of the preceding clauses and an adjustment tool, wherein the adjustment tool is adapted to adjust a distance between the first fixation point and the second fixation point against the magnetic force, in particular to bring the actuator (100) into a loaded state by adjusting the distance between the first fixation point and the second fixation point.
18. Use of the orthopaedic actuator (100) according to clauses 1 to 13 for an orthopaedic or orthodontic correction measure.

## Claims

1. An orthopaedic and/or orthodontic actuator (100, 110a, 110b) comprising
a first element (1) attachable to a first implant section (101),
a second element (2) attachable to a second implant section (102),
and a motion guide (3) coupling the first and second elements (1, 2)
and an actuator configured to control a relative movement of the first and second elements (1,2) from a first position and/or orientation to a second position and/or orientation along a predefined path,
wherein the actuator (100, 110a, 110b) comprises first and second magnets (15, 15a, 15b, 23, 23a, 23b) cooperating to urge the first and second elements (1, 2) along the predefined path.

2. The actuator (100, 110a, 110b) according to claim 1, comprising one or more springs (16, 16a, 16b) configured to urge the first and second elements (1, 2) along the predetermined path.

3. The actuator (100, 110a, 110b) according to claim 2, wherein the first and second magnets (15, 15a, 15b, 23, 23a, 23b) are arranged to provide an effective magnet force vector and wherein the one or more springs (16, 16a, 16b) are arranged to provide an effective spring force vector, wherein the magnet force vector and the spring force vector point in the same direction or opposite direction.

4. The actuator (100, 110a, 110b) according to claim 3, wherein the magnet force vector and the spring force vector coincide.

5. The actuator (100, 110a, 110b) according to any preceding claim, wherein the first element (1) comprises a first housing, and/or the second element (2) comprises a second housing;
and wherein at least one of, as the case may be,
the first housing accommodates the first magnet (15, 15a, 15b),
the second housing accommodates the second magnet (23, 23a, 23b), and
the first and/or second housing accommodates at least part of one or more springs (16, 16a, 16b) configured to urge the first and second elements along the predetermined path.

6. The actuator (100, 110a, 110b) according to any preceding claim, wherein the first and second elements (1, 2) are at least partially sleeved into each other to form a linear motion guide (3).

7. The actuator (100, 110a, 110b) according to any preceding claim, wherein the motion guide (3) is dimensioned such that it can withstand a tilt moment.

8. The actuator (100, 110a, 110b) according to any preceding claim, wherein the motion guide (3) has a low-friction coating.

9. The actuator (100, 110a, 110b) according to any preceding claim, wherein
the first element (1) comprises an at least partly soft magnetic first housing accommodating the first magnet (15, 15a, 15b), and/or
the second element comprises an at least partly soft magnetic second housing accommodating the second magnet (23, 23a, 23b).

10. The actuator (100, 110a, 110b) according to claim 9, wherein the at least partly soft magnetic first and/or second housing is dimensioned such as to remain below magnetic saturation in order to enhance the magnetic force of the actuator (100, 110a, 110b).

11. The actuator (100, 110a, 110b) according to any preceding claim, wherein the first element (1) comprises a first fixation point (10) for attaching the first element (1) to the first implant section (101) and/or the second element (2) comprises a second fixation point (20) for attaching the second element (2) to the second implant section (102).

12. The actuator (100, 110a, 110b) according to any preceding claim, wherein at least one of the first and second elements (1, 2) comprises an adjuster (4), e.g. an adjustment screw and possibly an adjustment plate (5), defining relative to a portion of, respectively, the first element (1) or the second element (2),
a position and/or orientation of the first or second magnet (15, 15a, 15b, 23, 23a, 23b), respectively, and/or
a position and/or orientation of one or more springs (16, 16a, 16b), if present.

13. The actuator (100, 110a, 110b) according to any preceding claim, wherein at least one of
- the first element (1) comprises a first magnet adjuster, e.g. an adjustment screw (4) and possibly an adjustment plate (5), defining a position and/or orientation of the first magnet (15, 15a, 15b) relative to at least part of the first element (1),
- the second element (2) comprises a second magnet adjuster, e.g. an adjustment screw (4) and possibly an adjustment plate (5), defining a position and/or orientation of the second magnet (23, 23a, 23b) relative to at least part of the second element (2), and
- the first and/or second element (1, 2) comprises a first and/or, respectively, second spring adjuster, e.g. an adjustment screw and possibly an adjustment plate, defining a position and/or orientation of the one or more springs (16, 16a, 16b) if present, relative to at least part of the first element (1) and/or, respectively, at least part of the second element (2), wherein optionally the first (second) magnet adjuster and, respectively, first (second) spring adjuster may be one first (second) adjuster.

14. The actuator (100, 110a, 110b) according to any preceding claim, wherein the first and/or second elements (1, 2) comprise at least one stop (13a, 22) defining an extreme relative position of at least part of the first and second elements (1, 2).

15. The actuator (100, 110a, 110b) according to any preceding claim, provided with an adjustment tool, e.g. a screw driver and/or a clamp retaining the actuator (100, 110a, 110b) in a loaded configuration.
